# EUROPEAN PATENT APPLICATION

(11) **EP 4 691 426 A1**
(43) Date of publication of application: **11.02.2026**
(21) Application number: 24193346.4
(22) Date of filing: 07.08.2024
(51) Int. Cl.: A61F 2/44

(54) **DEVICE FOR CORRECTION AND MAINTENANCE OF A POSITION OF SKELETAL ELEMENTS**

(71) Applicant: LfC sp. z o.o., 65-364 Zielona Gora (PL)
(72) Inventor: Ciupik, Lechoslaw Franciszek, 65-364 Zielona Góra (PL); Kierzkowska, Agnieszka Krystyna, 65-247 Zielona Góra (PL); Powchowicz, Pawel Ziemowit, 65-980 Zielona Góra (PL)
(74) Representative: PATERIS Patentanwälte PartmbB

(57) **Abstract**

A device for adapting and maintaining a position of a first bone and a second bone relative to each other is provided, the device comprising at least a first element with a proximal end and a distal end, wherein the first element comprises at least one helical thread-like coil extending from the sector of the distal end to the sector of the proximal end, and wherein the coil shows at least for a part of the thread closer to the distal end an increasing diameter starting from the distal end towards the proximal end, and at least one guide element is provided within the coil suitable for guiding the device upon placement of the device between two bones.

## Description

The present invention refers to a device for correcting a position of two bones and stabilizing the two bones in the position, particularly to an interosseous device.

Interosseous devices are known to be provided for the stabilization of a mutual position of two skeletal elements, namely bones.

The document US 8 142 504 B2 discloses a system for fixing a part to a bone element. The invention is essentially characterised by comprising two parts, namely an interbody implant with at least one aperture arranged in its upper part and a rod coiled in a spiral which like an awl pierces the bone during insertion by means of an installation tool, so that the first end of the rod moves alternately inside and outside the bone element and during its rotation the first end of the rod enters the aperture at least once. The helix used serves only to hold the installed implant in its target position and has no additional function related to correcting the reciprocal bone alignment.

The document US 2014 114 418 A1 discloses an implant assembly for the stabilisation of a facet joint. The two-piece implant has a U-shaped body comprising a rounded anterior end, an open posterior end, and two elongated arms terminating at the open posterior end. A spiral structure extending inside the body is inserted, which, as in the implant above, cuts through the bone like an awl and serves solely as a locking mechanism.

The document US 2017 156 879 A1 discloses spacer implants for bone fixation with an open spiral locking system and instrumentation for implant body implantation and fixation with a spiral lock. Here, as well, a spiral element is driven into the bone by piercing it. The implantation methods include following steps: attaching the implant spiral lock to the distal end of the elongated part of the vertebral body, where the elongated portion of the body encompasses the central core space of the spiral lock; positioning and attaching the selected implant to the implant insertion instrument; insertion of the implant and the spiral lock instrument assembly into the intervertebral space; insertion of the instrument, which transmits torsional forces to the face of the spiral lock, until the spiral lock is fully threaded into the adjacent bone and endplate. The spiral used in the device also only serves as a holding element for the implant in place.

The document US 8 197 513 B2 discloses a spinal implant having a wedge body with opposing bone-contacting surfaces, a distal end and a proximal end, a slot formed in each of the bone-contacting surfaces, such that each slot is in contact with a recess formed in at least a portion of the interior of the wedge body, and at least one screw pre-positioned in the recess of the wedge body such that threads formed on at least a portion of the screw protrude from the slots formed in the bone-contacting surfaces; A method comprising: surgically delivering the spinal implant to the facet joint in an intra-articular configuration; attaching the threads to opposite surfaces of the facet joint; and rotating the threads to move the wedge body into the facet joint. In this solution, the two parts of the implant are assembled together prior to insertion of the implant into the inter-articular space and cannot operate independently of each other.

The document WO 2022 216 566 A1 discloses a spinal implant comprising: an upper end plate; a lower end plate; a central body permanently positioned between the upper and lower end plates, the central body defining a first end, a second end opposite the first end and at least two sides; and multiple fixation slots, each of which is defined in part by a solid wall; wherein the spinal implant has a fixation opening between the upper end plate and the lower end plate; wherein each of the multiple of fixation slots extends from the second end of the central body to one of the upper end plate or the lower end plate, wherein at least one of the solid walls comprises: a receiving element configured to retain the bone screw in the one or more retaining slots; and a confirmation element configured to provide tactile feedback to a user when the bone screw has been fully inserted into the respective retaining slot.

The state of the art is related to devices suitable for stabilization of adjacent bones, particularly by implantation into vertebral bodies. However, it may be necessary to correct the mutual position of two bones prior to stabilization. There is a need for devices suitable for repositioning adjacent bones. It is a general objective of the present invention to provide an advantageous device facilitating both repositioning and stabilizing the mutual position of two adjacent bones.

The objective is solved by a device according to claim 1. Further advantageous embodiments of the invention are contained in the dependent claims, the figures and the examples.

A first aspect of the invention is related to a device for adapting and maintaining a position of a first bone and a second bone relative to each other, the device comprising at least a first element with a proximal end and a distal end, wherein
the first element comprises at least one thread-like helical coil extending from the sector of the distal end to the sector of the proximal end, and wherein the coil shows at least at a part a gradually increasing diameter starting from the distal end towards the proximal end, and
the first element comprises means suitable for guiding the device upon placement of the device between the first bone and the second bone.

The inventive device is advantageous because in addition to a fixation of adjacent bone it facilitates a repositioning of adjacent bones. It can be used for correction and maintenance of the position of skeletal elements. The invention facilitates not only interbone fixation, but also correction of a reciprocal bone alignment.

The increasing diameter of the coil is advantageous because it facilitates an effective placement of the device into a bone or in a position between two bones, respectively. The design with the thread, and the small diameter at the distal end, which is formed as a tip, facilitates a functioning similar to a wood screw by driving it into the bone. By positioning the decice between two adjacent bones the distance of the bones can be adjusted by screwing the device into a position where a certain diameter of the device facilitates the distance. The mutual position of the bones can be changed at a later point of time, if desired, by turning the device further into the bone or outbound of the bone, so that the diameter corresponds to a certain distance of the bones.

The means for guiding the first element are suitable for positioning the element in a desired position and final interaction with the adjacent bones. The means can comprise the entire first element or features of the first element designed for adaptation of instruments which are part of the first element or supplementary components.

The first element as such is usable as a stand-alone device. It is yet in the essence of the invention that the first element can be combined with other elements. Thus, different configurations of the inventive device are possible depending on the planned application.

In a preferred embodiment, the device further comprises at least a second element comprising at least a first bone contacting surface at its upper side, at least a second bone contacting surface at its lower side and at least one open recess in the surface of the first and/or second bone contacting surface, wherein the recess comprises slits arranged inside the recess and has a size and geometry corresponding to the size and geometry of the thread-like coil of the first element, so that the first element can fittingly interact with the second element, so that upon final positioning of the device in a space between the first bone and the second bone the first element is partly stabilized in at least one bone and partly in the second element by effect of the coil.

The first and second elements are independent parts of the inventive device, yet they are preferably provided in order to cooperate in order to facilitate a repositioning and a fixation of two adjacent bones, particularly of vertebral bodies. The second element is formed to fit in the interosseous space between two bones, and the first element is formed to move the two bones into a certain position, which is possible by moving the first element into the second element, thereby pushing one bone into a position upward from the device.

Preferably the helical coil comprises at least one cutting element and/or at least one anchoring element. Ideally more than one cutting element or anchoring element are provided, respectively. The cutting elements facilitate advantageously the movement of the first element into the bone material by moving the helical coil comparable to a screw; the helical coil thus bears a similarity to a thread. The cutting elements can e.g. be formed as blades.

The anchoring elements facilitate an embedding of the first element in the bone material, promoting a fusion of the material of the first element to the bone material. The anchoring elements can e.g. be formed as protrudings from the material of the helical coil.

In a preferred embodiment of the invention, the first element comprises a core stretching from the distal end to the proximal end of the first element, around which the helical coil is arranged in a longitudinal direction. The core advantageously provides a stabilizing and guiding function. The core can be formed as a shaft or a cone. Preferably, the core is formed as a shaped cone. The shaped cone advantageously causes the helical coil to cut into the bone and/or translate the bone directily or indirectly though the second element.

Preferably a section of the helical coil shows notches of the helical coil such that the material of at least one turn of the thread is formed as at least two vanes radially extending from the core. This structure advantageously promotes a forwardly directed movement of the first element, along with providing an anchoring function, as the recessed material allows bone material to grow around or into the helical coil after implantation. In a preferred embodiment, the vanes are basically mutually opposite. This means that in a pair of vanes, the two vanes are preferably arranged on basically opposing sides of the core.

Preferably the core comprises an axial hole extending from the proximal end of the core. The axial hole advantageously facilitates a precise guidance of the first element over a guiding element, e.g. a Kirschner wire, during an implantation process. Furthermore, it facilitates transport of bone graft or cement during an implantation process.

In a further preferred embodiment of the invention the first element has means at its proximal end for connecting to other devices. The means can be formed as a socket. The means can have forms for accommodating a tool which is formed to drive the implant, wherein the means for accommodating the tool and the tool have complementary forms, so that the tool is positively insertable into the means. The means can also be formed to be connectable to other devices, e.g. implants.

In a further preferred embodiment of the invention the first element comprises a resistive shelf at its proximal end. Preferably the first element terminates in the resistive shelf. The resistive shelf can advantageously provide a cooperation with the bone, such as to provide a support of the device at the bone.

In a further preferred embodiment, there is at least one channel-likestructure formed in the surface of the coil suitable for transporting bone material. The channel-like structure can be formed as a groove running along the helical coil, thereby supporting the transport of bone material from the distal end towards the proximal end. This again advantageously promotes the integration of the first element into the bone material.

In a preferred embodiment, a possible angle of interaction between the first and the second element can be set between 0 and 90°. This is advantageous because it makes possible a movable cooperation of any necessary adaptation of the mutual position of two bones. With other words the two elements can be set parallel to each other or angled between 0 and 90°. The angle is defined by the central axis of the first element, which is set at a desired angle to the second element.

In a further preferred embodiment, the second element comprises at least one rib-like structure suitable for guiding the movement of the first element relative to the second element. In addition to the guiding function, the rib-like structure can help to maintain a certain angle between the first and the second element. For this, the rib-like structure is provided to facilitate the postion of the first element at a certain angle relative to the second element. Thus, the angle between the first and second element has to be known upon preparation of the second element.

In a further preferred embodiment, the second element comprises a part wherein the material is branched into at least two arms. This design facilitates a modification of the three-dimensional appearance of the second element, which promotes advantageous adaptation of the device to the geometry of an entire interosseous space. With other words, the body can be provided at least with two arms in order to increase the bone contact surface and/or to trigger distraction of the bones.

In a further preferred embodiment, the second element comprises three-dimensional structures suitable for promoting fusion of the device to bone material. Thereby an ingrowth and thus a stable implantation of the device to one or two bones is promoted.

In a further preferred embodiment, the first element and the second element comprise means for locking the elements in a fixed position relatively to each other. This is particularly advantageous because the first element can be fixed in a final position relative to the second element.

In a further preferred embodiment the device is an interosseous device configured to engage two vertebral bodies. The device can also be configured for engaging any other group of bones.

A second aspect of the invention is related to a method for correcting and maintaining a position of skeletal elements, the method comprising the steps of
Evaluation of the position of a first and second adjacent bones planned for correction and stabilization, and preparation of the interbone space for a device according to the invention,
Selecting a geometrically appropriate second element and placing it between the first bone and second bone,
Insertion of the first element into the second element at a predetermined angle and anchoring of the first element in the first bone and/or the second bone,
Stabilization of the device by locking the position of the first element and the second element.

The inventive method is advantageous for the same reasons as the inventive device. After implantation, the implant can preferably further be stabilized by addition of bone graft or cement medium.

Embodiments of the invention are described in conjunction with the accompanying figures.
- Figure 1: shows an embodiment of a device according to the present invention.
- Figure 2: shows a cross section of the device according to Fig. 1 as inserted between two bones.
- Figure 3: shows a perspective view on an embodiment of a first element of the inventive device.
- Figure 4: shows an alternative perspective view of the first element of Fig. 3.
- Figure 5: shows a cross section through an alternative embodiment of the inventive device.
- Figure 6: shows a perspective view on an embodiment of a second element of the inventive device.
- Figure 7: a flow chart of a method according to the present invention.

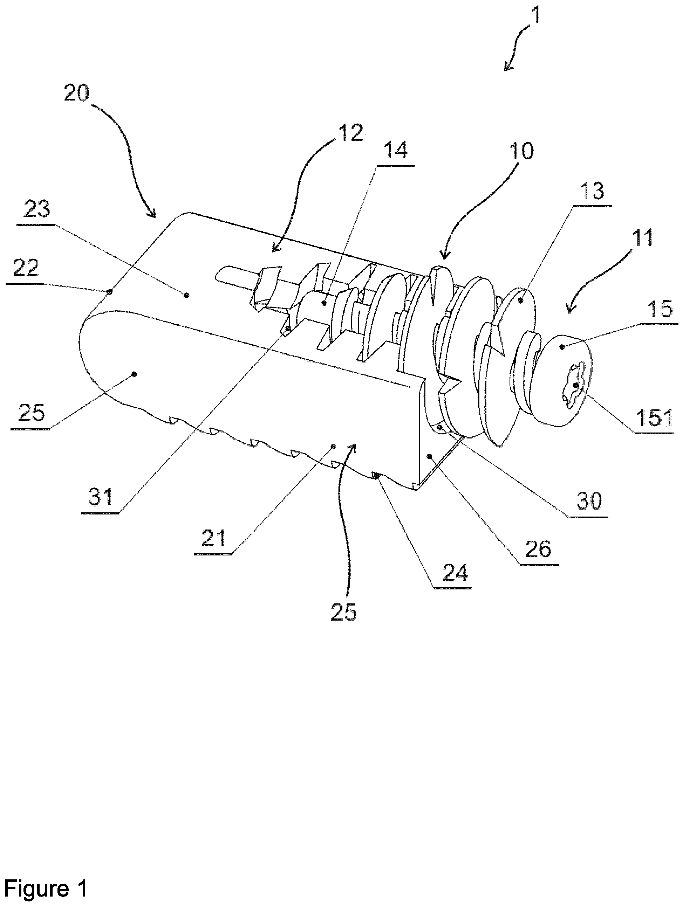

An embodiment of an inventive device 1 according to Fig. 1 comprises a first element 10 and a second element 20. The named elements are independent from each other and can be used as stand-alone devices. In the embodiment according to Fig. 1, the first element 10 and the second element 20 are shown to act in cooperation.

The first device 1 has a proximal end 11 and a distal end 12. The first device 10 comprises a helical coil 13, which is formed and functioning like a thread. The helical coil 13 is running from close to the distal end 12 to close to the proximal end 11 of the first element 10. Up to about a central section of the first element 10, the diameter of the helical coil is increasing from the distal end 12 towards the proximal end 11. From the central section of the helical coil 13 to about the end of the helical coil 13 near the proximal end 11, the diameter of the helical coil 13 is constant.

The helical coil 13 is arranged to coil around a core 14. The core 14 is formed as a rotating shaft, to which a torque can be applied via an appropriate tool in order to turn the helical coil 13 upon implantation of the device 1 into an interosseous space. Here, the core 14 is stretching from the proximal end 11 to the distal end 12 of the first element 10. In an alternative embodiment, the first element 10 can be composed of only the helical coil 13 itself, without a core.

At the proximal end 11 the first device 10 has a socket 15. The socket 15 shows a recess opening 151 which shows a special form which is provided to accommodate a tool with an appropriate form to fit in the recess. As mentioned above, the tool would be inserted to apply a force to the first element 10, particularly a torque. Alternatively, other devices could be connected to the first element 10 via the socket 15.

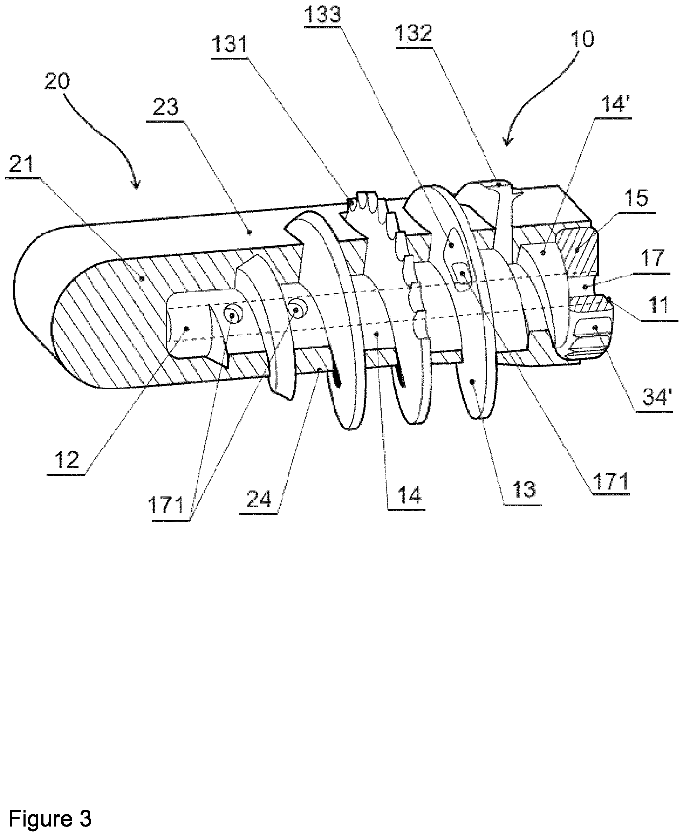

The second element 20 comprises a body 21. The overall form of the body 21 is similar to a cuboid, however with a roundish form of the front side 22, which corresponds to the distal end 12 of the first element 10. The body 21 has an upper side 23 and a lower side 24. The body has further two lateral sides 25 and a back side 26. The upper side 23 shows a recess 30, which is open towards the back side 26 and closed towards the front end 22. Alternatively, a recess can be formed at the lower side 24, or on both the upper side 23 the lower side 24 (see Fig. 3). In the recess 30 guiding slits 31 are provided with a geometry which corresponds to the forms of the turn of the helical coil 13.

The material of the device 1 can consist of suitable materials known to the expert. The first and second element 10, 20 can consist of a metal, e.g. titanium, or an appropriate metal alloy, or of various types of polymers, including biomaterials.

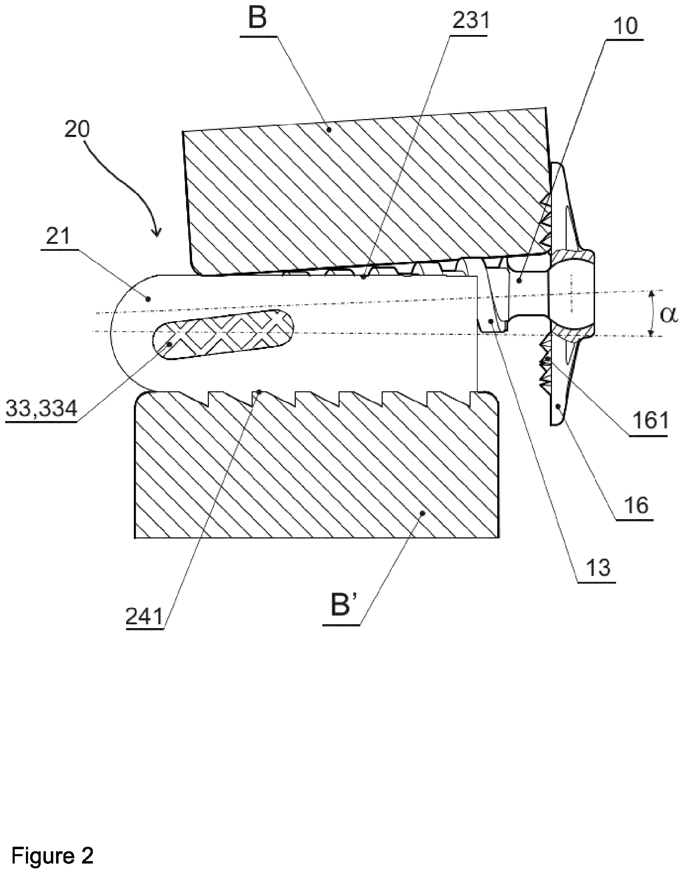

In Fig. 2 a cross section of the inventive device 1 as inserted in the interosseous space between two bones is shown. The body 21 of the second element 20 is positioned between a first bone B, which is in contact with a first bone contacting surface 231 at the upper side 23, and a second bone B', which is in contact with a second bone contacting surface 241 at the lower side 24.

The first element 10 is positioned in the recess 30 of the body 21, so that the helical coil 13 is in contact with the body 21 and the second bone B'.

The first element 10 and the second element 20 can cooperate movably at a certain angle α, which is in Fig. 2 about 5°. The angle can be set between 0 and 90 °.

The first element 10 as shown in Fig. 2 bears a resistive shelf 16 at its proximal end 11. The resistive shelf 16 is there to support the device 1, particularly the first element 10, at the first bone B. It bears protrusions 161 which are in contact with the first bone B, thus securing the position of the first element 10 and promoting its fusion to the first bone B.

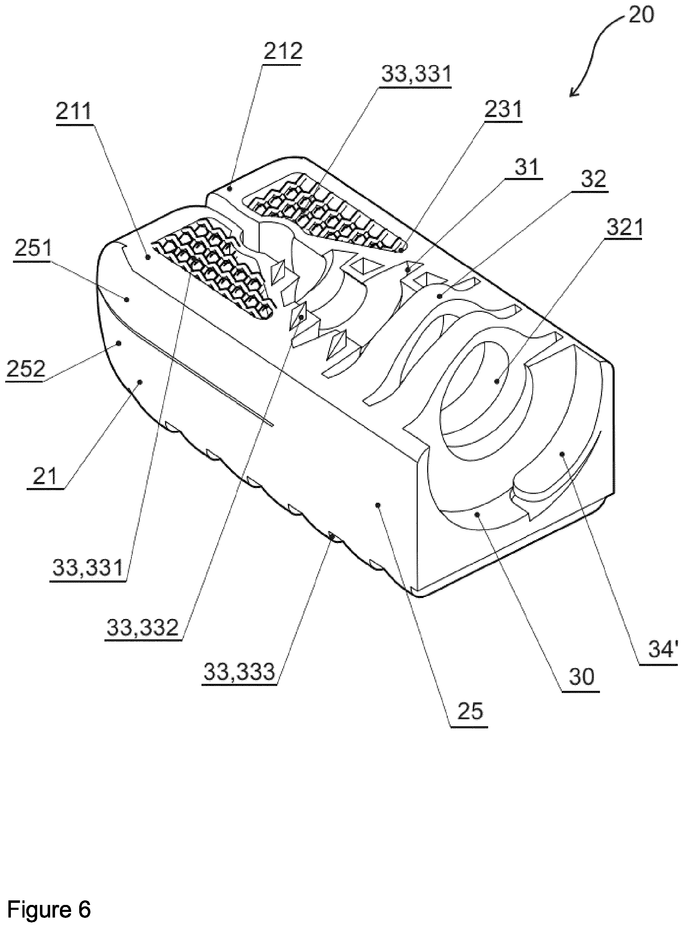

Further in Fig. 2, the second element 20 shows three-dimensional structures 33 suitable for promoting fusion of the device 1, particularly of the second element 20, to the bone material. The three-dimensional structures 33 in Fig. 2 are formed as a truss-like structure 334. Further embodiments of three-dimensional structures 33 in the second element 20 are shown in Fig. 6. In an embodiment of the device 1 according to Fig. 3 the helical coil 13 of the first element 10 comprises cutting elements 131 in the central turn, wherein the coil 13 is formed like a saw. These cutting elements 131 support the movement of the first element 10 into a bone material by screwing and sawing of the helical coil 13. The cutting elements 131 also promote a fusion of the material of the first element 10 to the bone material. Further elements promoting an embedding of the first element 10 in the bone are anchoring elements 132 and channel-like structures 133, which can be formed as grooves.

Further in Fig. 3, the first element 10 shows an axial hole 17 starting at the proximal end 11. The axial hole 17 may be blind, i.e. be formed as a dead end within the core 14, or be formed as a through hole with a second opening at the distal end 12. Further, the core 14 is formed as a shaped cone 14' near the proximal end 11.

Additional channels 171 are formed both in the coil 13 and in the core 14 in order to connect the surface of the first element 10 to the axial hole 17. As shown here, the additional channels 171 have openings in the channel-like structures 133 and in the core 14. Alternatively, the openings can be formed only in the channel-like structures 133 or in the core 14, or additionally or alternatively in other parts of the first element 10. The additional channels 171 are provided in order to promote the fusion of the first element 10 to the material of the bones it is placed into.

As mentioned above, Fig. 3 shows that the recess 30 in the second element 20 is formed on both the upper side 23 and the lower side 24. The first element 10 is projected bilaterally over the upper and lower sides 23, 24 of the second element 20.

Further in Fig. 3, the first element 10 has a first locking element 34 its proximal end 11, which is provided in order to interlock with a matching locking element 34' of the second device 20 (see Fig. 6).

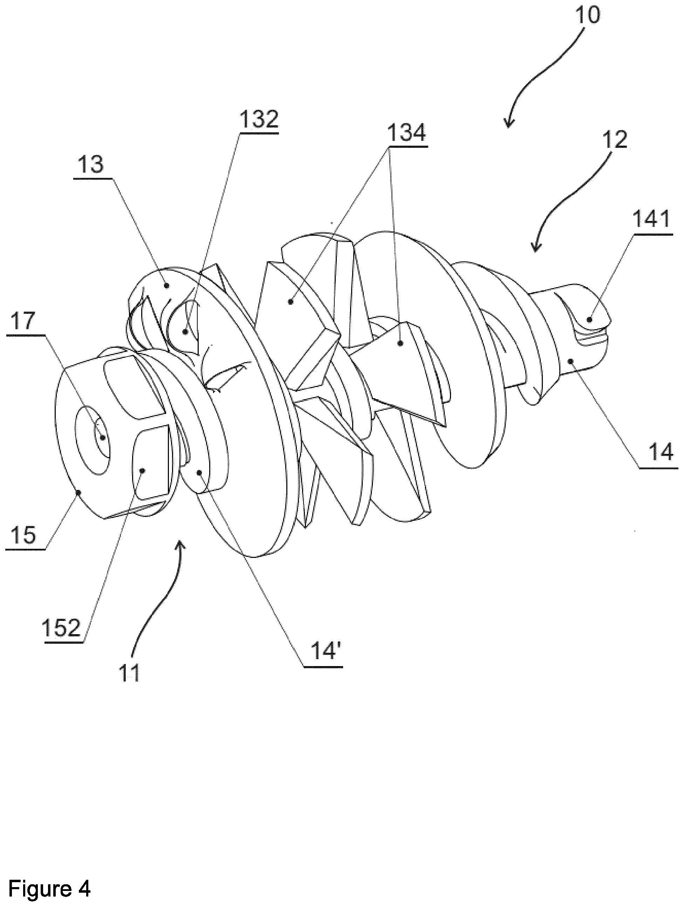

In an embodiment of the first element 10 according to Fig. 4 the helical coil 13 shows additional features. At the distal end 12, the ending portion of the core 14 is formed to bear at least one second locking element 141. The locking element 141 is designed to match with a counterpart in the second element 20 (not shown) in order to interlock the first and second elements 10, 20 in a fixed arrangement.

Further in Fig. 4, the helical coil 13 is formed to have recesses so that the thread shows a form of vanes 134 for two turns of the thread. The vanes 134 of the corresponding turns are in a mutual overlapping position.

Further in Fig. 4, anchoring elements 132 are formed at the ending of the helical coil 13 at the proximal end 11. The anchoring elements 132 are here formed at the outside of the thread pointing towards the proximal end 11.

Further in Fig. 4, the socket 15 shows indentations 152 at its outer circumference. The form and number of the indentations 152 (namely 6) have the appearance of an allen key and provide a possibility for a device of a tool with a matching geometry to be effectively connected.

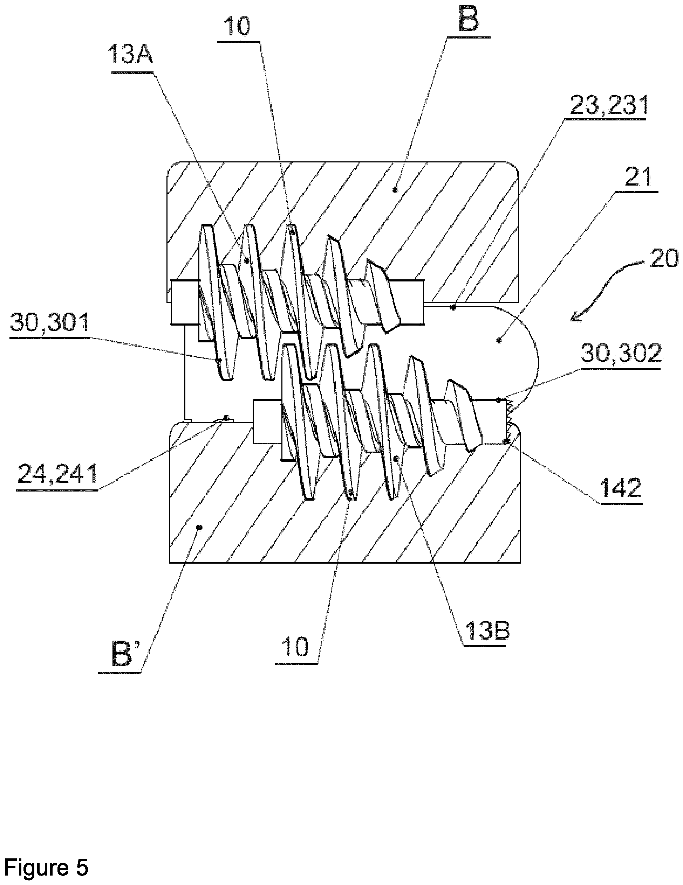

Fig. 5 shows a cross section through an embodiment of the inventive device 1 with two first elements 10 joined with one second element 20. Here the second element 20 shows two recesses 30: a first recess 301 in order to interact with a first helical coil 13A at the first bone contacting surface 231, and a second recess 302 in order to interact with a second helical coil 13B at the second bone contacting surface 241. The helical coil 13A is driven into the first recess 301 of the upper side 23, so it is in contact with the second device 20 and the first bone B. The helical coil 13B is driven into the second recess 302 of the lower side 24, so it is in contact with the second device 20 and the second bone B'.

As it is shown at the distal end of the first element 10 bearing the second helical coil 13B, further teeth-like cutting elements 142 are provided its distal end 12. The teeth-like cutting elements 142 are provided in order to cut through the bone material.

In an embodiment of the second element 20 according to Fig. 6 two rib-like structures 32 are provided at the end of the recess 30 which corresponds to the proximal end 11 of the first element 10. The rib-like structures 32 are suitable for guiding the movement of the first element 10 relatively to the second element 20. The first element 10 is inserted into the rib-like structures 32 via the opening 321.

Further in Fig. 6, the second element 20 shows three-dimensional structures 33 suitable for promoting fusion of the device 1, particularly of the second element 20, to the bone material. The three-dimensional structures 33 as shown in Fig. 6 are provided in three different embodiments. They are shown to be in the form of a honeycomb structure 331 at the portion toward the front end, and in the form of pyramid-like protrusions 332 in the more central portion of the first bone-contacting surface 231. Furthermore, three-dimensional structures 33 in the form of a serrated structure 333 are shown at the second bone contacting surface 241.

Further in Fig. 6, in the portion towards the back side 26, the second element 20 has a locking element 34'. The locking element 34' of the second element 20 is configured to match with the first locking element 34 at the proximal end 11 of the first element 10.

Further in Fig. 6, in the body 21 comprises a first arm 211 and a second arm 212. The arms 211, 212 are interconnected and movable in order to spread and cover a bigger volume of an interosseous space, respectively, as compared to a body 21 without arms (Fig. 1). The lateral sides of the second element 20 can be split into an upper lateral side 251 and a lower lateral side 252. These are also provided in order to extend the device 1 and to cover a bigger volume of interosseous space.

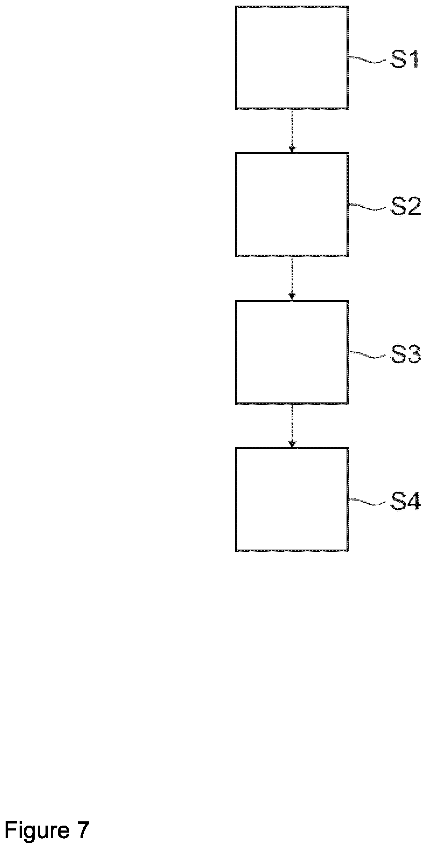

In step S1 of a method for correcting and maintaining a position of skeletal elements according to Fig. 7, a position of a first bone B and second adjacent bone B' planned for correction and stabilization is evaluated. The interosseous space is prepared for the insertion of a device 1, which comprises a first element 10 and a second element 20. In a second step S2, a geometrically appropriate second element 20 is selected and placed it between the first bone B and second bone B'. In a third step S3, the first element 10 is inserted into the second element 20 at a predetermined angle and anchoring of the first element in the first bone and/or the second bone. The first element 10 is driven into the second element 20 by applying a force driving the helical coil 13. The angle is predetermined depending on a planned fixation and or correction of a bone position, such that the first element 10 is contacting the second device 20 and the first bone B, thereby pushing the first bone B until the determined angle is reached so that the device 1 holds and fixes the two bones B, B' in a desired position.

In a fourth step S4, the device 1 is stabilized by locking the position of the first element 10 and the second element 20. The device 1 can further be stabilized by addition of bone graft or cement medium, which promote fusion of the device 1 to the bones.

### List of reference signs

- 1: device
- 10: first element
- 11: proximal end
- 12: distal end
- 13: helical coil
- 13A: first helical coil
- 13B: second helical coil
- 131: cutting element of the coil
- 132: anchoring element of the coil
- 133: channel-like structure
- 134: vane
- 14: core
- 14': shaped cone
- 141: second locking element of the first element
- 142: cutting element of the core
- 15: socket
- 151: recess opening
- 152: indentations
- 16: resistive shelf
- 161: protrusions
- 17: axial hole
- 171: additional channel
- 20: second element
- 21: body
- 211: first arm
- 212: second arm
- 22: front side
- 23: upper side
- 231: first bone contacting surface
- 24: lower side
- 241: second bone contacting surface
- 25: lateral side
- 251: upper lateral side
- 252: lower lateral side
- 26: back side
- 30: recess
- 301: first recess
- 302: second recess
- 31: guiding slit
- 32: rib-like structure
- 321: opening
- 33: three-dimensional structure
- 331: honeycomb-like structure
- 332: pyramid-like structure
- 333: serrated structure
- 334: truss-like structure
- 34: first locking element of the first element
- 34': locking element of the second element
- B: first bone
- B': second bone

## Claims

1. Device (1) for adapting and maintaining a position of a first bone (B) and a second bone (B') relative to each other, the device (1) comprising at least a first element (10) with a proximal end (11) and a distal end (12), wherein
the first element (10) comprises at least one thread-like helical coil (13) extending from the sector of the distal end (12) to the sector of the proximal end (11), and wherein the coil (13) shows at least at a part a gradually increasing diameter starting from the distal end (12) towards the proximal end (11), and
the first element (10) comprises means suitable for guiding the device (1) upon placement of the device (1) between the first bone (B) and the second bone (B').

2. Device (1) according claim 1, **characterized in that** the device (1) further comprises at least a second element (20) comprising at least a first bone contacting surface (231) at its upper side (23), at least a second bone contacting surface (241) at its lower side (24), and at least one open recess (30) in the first bone contacting surface (231) and/or second bone contacting surface (241),
wherein the recess (30) comprises guiding slits (31) arranged inside the recess (30) and has a size and geometry corresponding to the size and geometry of the helical coil (13) of the first element (10), so that the first element (10) can fittingly interact with the second element (20), so that upon final positioning of the device (1) in a space between the first bone (B) and the second bone (B') the first element (10) is partly stabilized in at least one bone and partly in the second element (20) by effect of the coil (13).

3. Device (1) according to any of the foregoing claims, **characterized in that** the helical coil (13) comprises at least one cutting element (131) and/or at least one anchoring element (132).

4. Device (1) according to any of the foregoing claims, **characterized in that** the first element (10) comprises a core (14) stretching from the distal end (12) to the proximal end (11) of the first element (10), around which the helical coil (13) is arranged in a longitudinal direction.

5. Device (1) according to claim 4, **characterized in that** the core (14) is formed as a shaped cone (14').

6. Device (1) according to claim 4 or 5, **characterized in that** a section of the helical coil (13) shows notches of the helical coil (13) such that the material of at least one turn of the thread of the helical coil (13) is formed as at least two vanes (134) radially extending from the core (14).

7. Device (1) according to any of claims 4 to 6, **characterized in that** the core (14) comprises an axial hole (17) extending from the proximal end (11) of the core (14).

8. Device (1) according to any of the foregoing claims, **characterized in that** the first element (10) has means at its proximal end (11) for connecting to other devices.

9. Device (1) according to any of the foregoing claims, **characterized in that** the first element (10) comprises a resistive shelf (16) at its proximal end (11).

10. Device (1) according to any of the foregoing claims, **characterized in that** at least one channel-like structure (133) is formed in the surface of the coil (13) suitable for transporting material.

11. Device (1) according to claim 1 or 2, **characterized by** a possible angle of interaction between the first element (10) and the second element (20) between 0 and 90°.

12. Device (1) according to one of the foregoing claims, **characterized in that** the second element (20) comprises at least one rib-like structure suitable (32) for guiding the movement of the first element (10) relative to the second element (20).

13. Device (1) according to one of the foregoing claims, **characterized in that** the second element (20) comprises a part where the material is branched into at least two arms (211, 212).

14. Device (1) according to one of the foregoing claims, **characterized in that** the first element (10) and the second element (20) comprise means for locking the first and second elements (10, 20) in a fixed position relatively to each other.

15. Method for correcting and maintaining a position of skeletal elements, the method comprising the steps of
Evaluation of the position of a first and second adjacent bones (B, B') planned for correction and stabilization, and preparation of the interbone space for a device (1) according to any of claims 1 to 14,
Selecting a geometrically appropriate second element (20) and placing it between the first bone (B) and second bone (B'),
Insertion of a first element (10) into the second element (20) at a predetermined angle and anchoring of the first element (10) in the first bone (B) and/or the second bone (B'),
Stabilization of the device (1) by locking the position of the first element (10) and the second element (20).
